(19) Europäisches Patentamt
European Patent Office
Office européen des brevets

(11) Veröffentlichungsnummer: **0 308 568**
**A1**

(12) # EUROPÄISCHE PATENTANMELDUNG

(21) Anmeldenummer: **87810559.2**

(22) Anmeldetag: **25.09.87**

(51) Int. Cl.⁴: **C07D 498/08 , A61K 31/395 , //(C07D498/08,307:00,267:00)**

(43) Veröffentlichungstag der Anmeldung: **29.03.89 Patentblatt 89/13**

(84) Benannte Vertragsstaaten: **AT BE CH DE ES FR GB GR IT LI LU NL SE**

(71) Anmelder: **CIBA-GEIGY AG**
**Klybeckstrasse 141**
**CH-4002 Basel(CH)**

(72) Erfinder: **Traxler, Peter, Dr.**
**Bündtenring 5**
**CH-4124 Schönenbuch(CH)**
Erfinder: **Müller, Klaus, Dr.**
**Fürstensteinhof 7**
**CH-4107 Ettingen(CH)**
Erfinder: **Kump, Wilhelm, Dr.**
**Friedrich-Oser-Strasse 10**
**CH-4105 Biel-Benken(CH)**

(54) **Diacylderivate von 4-( Trialkylbenzyl)-piperazinyl-Verbindungen.**

(57) Verbindungen der Formel

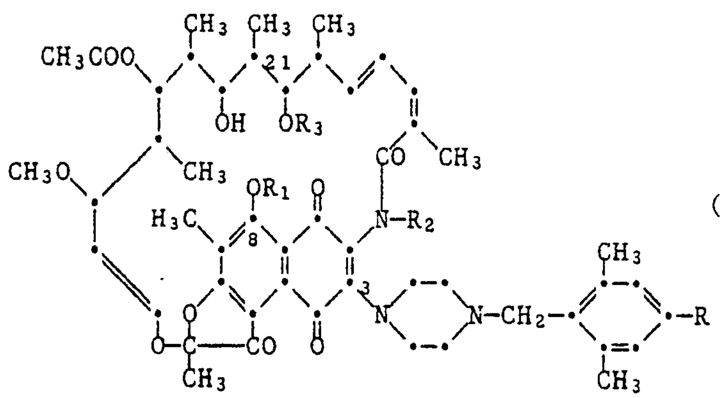

(I)

worin R Niederalkyl bedeutet, $R_1$ Triniederalkylmethylcarbonyl darstellt, und einer der Reste $R_2$ und $R_3$ Triniederalkylmethylcarbonyl und der andere Wasserstoff bedeuten, und ihre Salze zeigen hypolipidämische Eigenschaften.

EP 0 308 568 A1

## Diacylderivate von 4-(Trialkylbenzyl)-piperazinyl-Verbindungen

Gegenstand der vorliegenden Erfindung sind neue Diacylderivate von Rifamycinen, die in 3-Stellung einen substituierten 1-Piperazinyl-Rest tragen, und zwar 8-O,N- und 8-O,21-O-Diacylderivate von Rifamycin S-Verbindungen der Formel

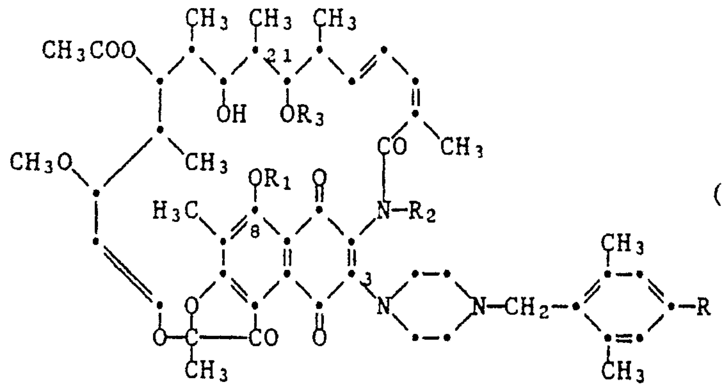

(I)

worin R Niederalkyl bedeutet, $R_1$ Triniederalkylmethylcarbonyl darstellt, und einer der Reste $R_2$ und $R_3$ Triniederalkylmethylcarbonyl und der andere Wasserstoff bedeuten, ihre Salze, sowie Gemische von isomeren Verbindungen der Formel I bzw. deren Salzen.

Die Erfindung betrifft ferner auch Verfahren zur Herstellung von Verbindungen der Formel I und ihrer Salze, diese enthaltende pharmazeutische Präparate, sowie die Verwendung dieser Verbindungen und Präparate.

Die im vorliegenden Fall angewandte Numerierung bezieht sich auf die z.B. im USA Patent Nr. 4 005 077 verwendete Numerierung.

Niederalkyl R enthält vorzugsweise bis und mit 4 Kohlenstoffatome und ist z.B. Ethyl, Propyl, i-Propyl, n-Butyl, Isobutyl oder tert-Butyl, in erster Linie aber Methyl.

Niederalkylreste in einem Triniederalkylmethylcarbonylrest enthalten üblicherweise bis und mit 4, vorzugsweise bis und mit 2 Kohlenstoffatome und in erster Linie ein Kohlenstoffatom, und bedeuten u.a. n-Propyl, Isopropyl, n-Butyl oder tert.Butyl, insbesondere Ethyl und in erster Linie Methyl.

In den Verbindungen der Formel I haben die Reste $R_1$ und $R_2$ oder $R_3$ vorzugsweise die gleiche Bedeutung.

Nicht acylierte Rifamycin S-Verbindungen, die in 3-Stellung eine in 4-Stellung substituierte Piperazinogruppe aufweisen, sind bekannt. So werden z.B. im US-Patent 4 005 077, vor allem im Beispiel 77, solche Rifamycin-Derivate erwähnt, die in 4-Stellung einen Benzylrest enthalten, der im aromatischen Teil durch ein oder mehrere Alkylreste mit 1 bis 6 Kohlenstoffatomen substituiert sein kann, wie 3-(4-Benzyl-1-piperazinyl)-rifamycin SV, 3-[4-(4-Methylbenzyl)-1-piperazinyl]-rifamycin SV, 3-[4-(2-Methylbenzyl)-1-piperazinyl]-rifamycin SV, 3-[4-(3-Methylbenzyl)-1-piperazinyl]-rifamycin SV, 3-[4-(4-Isopropylbenzyl)-1-piperazinyl]-rifamycin SV, 3-[4-(2,3-Dimethylbenzyl)-1-piperazinyl]-rifamycin SV und 3-[4(4-tert-Butyl-benzyl)-1-piperazinyl]-rifamycin SV. Weitere Verbindungen dieses Typs, die insbesondere einen 4-(2,6-Dimethyl-4-alkyl-benzyl)-piperazinrest aufweisen, sind z.B. in der PCT-Anmeldung Publ. Nr. WO 87/02361 beschrieben. Diese Verbindungen zeichnen sich durch eine antibiotische, vor allem antituberkulöse Wirkung aus, die an Mäusen oder Ratten in vivo nachgewiesen werden kann, und die, bezogen auf das Wirkungsspektrum, etwa derjenigen des bekannten antituberkulösen Mittels Rifampicin entspricht.

Die Diacyl-Verbindungen der vorliegenden Erfindung dagegen weisen, wenn überhaupt, eine nur marginale antibiotische Wirkung auf. Ueberraschenderweise besitzen sie jedoch eine lipidsenkende Wirkung, die in Tierversuchen, vorzugsweise an Säugetieren, z.B. an Ratten, nachgewiesen werden kann. So kann die Senkung von "very low density"-, "low-density"- bzw. "high density"-Lipoproteinen (VLDL, LDL bzw. HDL) im Serum in zwei Testanordnungen, und zwar in genetisch hypercholesterolämischen männlichen Ratten (Anordnung A) und normolipämischen Ratten beiden Geschlechts (Anordnung B) gezeigt werden.

Albino-Ratten mit einem Körpergewicht von 180-240 g, die freien Zugang zu Standard-Rattenfutter und Trinkwasser haben, werden verwendet, und zwar in Anordnung A Sprague Dawley Abkömmlinge des

Stammes Tif:RAI und in Anordnung B Tiere des Wistar-Stammes IVa-WI. Die Testverbindung wird in einer Polyethylenglykollösung (mittleres Molekulargewicht von 400) oral an Gruppen von 8 bis 10 Ratten verabreicht, in der Anordnung A einmal täglich um 8 Uhr vormittags während drei aufeinanderfolgenden Tagen und zweimal, um 8 Uhr und 16 Uhr, am vierten Tag, oder im Falle der Anordnung B täglich während 5 aufeinanderfolgenden Tagen. Im Experiment A werden die Tiere 16 Stunden und im Experiment B zwei Stunden nach der letzten Gabe durch Ausblutenlassen vom Hals unter Anästhesie mit Ether getötet. Während 16 Stunden vor ihrem Tod erhalten die Tiere keine Nahrung mehr. Zum vereinigten Serum von 2 bis 3 Ratten fügt man eine 0,05%ige wässrige Ethylendiamintetraessigsäure-Lösung und eine 0.01%ige wässrige Thiomersal-Lösung. Die Serumlipoproteine werden unter Verwendung einer Ultrazentrifuge durch 24-stündiges Zentrifugieren bei 78 000 g, 78 000 bzw. 109 000 g in Salzlösungen mit den Dichten 1,006, 1,040 bzw. 1,21 getrennt und auf Gehalt von Cholesterin und Triglyceriden enzymatisch mit Hilfe der z.B. von Miles (Lausanne, Schweiz) bzw. Böhringer (Mannheim, Bundesrepublik Deutschland) gelieferten Testsysteme analysiert.

Die Ermittlung der antibiotischen Wirkung erfolgt z.B. einerseits in vitro durch die Bestimmung der mittleren effektiven Konzentration $EC_{50}$ für die Hemmung der RNA-Polymerase von Escherichia coli, sowie der minimalen Hemmkonzentration MIC ("minimum inhibitory concentration") im konventionellen Plattentest, andererseits in vivo an infizierten Mäusen und Ratten durch die Bestimmung von $ED_{50}$ (effektive Dosis, die für 50 % der Versuchstiere lebenserhaltend ist). Als Mikroorganismen werden für den vorliegenden Zweck insbesondere Mycobacterium tuberculosis TB $H_{37}Rv$ und Staphylococcus aureus verwendet. Bei Verbindungen mit lipidsenkender Indikation wird eine antibiotische Wirksamkeit als nachteilig erachtet, da sie, insbesondere bei langfristiger Verabreichung, zur Bildung von gegen Antibiotika resistenten Stämmen von Mikroorganismen führen kann.

In den oben beschriebenen Testmethoden weisen die erfindungsgemässen Verbindungen sowohl bei einmaliger wie auch wiederholter Verabreichung im Dosisbereich von etwa 3 bis etwa 50 mg/kg/Tag eine signifikante hypolipidämische Wirksamkeit auf; dagegen sind sie in den oben erwähnten Tests frei von nennenswerter antibiotischer Aktivität.

So kann z.B. gezeigt werden, dass, je nach Versuchsanordnung, die minimale effektive Dosis des 8-O,N-Dipivaloyl-3-[4-(2,4,6-trimethylbenzyl)-1-piperazinyl]-rifamycin S bei einmaliger Verabreichung bei etwa 3 bis etwa 10 mg/kg liegt, und durch eine wiederholte Verabreichung von täglich 30 mg/kg eine 50-70%ige Senkung der "LDL-Fraktion" erreicht werden kann. Dabei hat die Verbindung praktisch keine antibiotische Wirksamkeit; eine $EC_{50}$ für die Inhibition der RNA-Polymerase wird mit 100 µg/ml noch nicht erreicht, und die MIC für verschiedene pathogene Stämme von Staphylococcus aureus liegt bei etwa 64 µg/ml. Solche Werte sind etwa 1000-fach höher als Konzentrationen, die für einen entsprechenden Effekt normalerweise erforderlich sind. Auch in vivo unter Verwendung von mit Staphylococcus aureus infizierten Mäusen erweist sich die Verbindung bei einer Einzeldosis von 200 mg/kg als antibiotisch unwirksam. Analoge Resultate werden auch mit der entsprechenden 8-O,21-O-Dipivaloyl-Verbindung gefunden.

Insbesondere dank ihrer LDL-senkenden Wirkung können die erfindungsgemässen Verbindungen, z.B. als Hypolipidämika zur Behandlung von Hyperlipidämien, hauptsächlich der Typen IIa und IIb, und Atherosclerose bei Vorliegen von Hyperlipiproteinämie als Risikofaktor verwendet werden.

Einen bevorzugten Gegenstand der vorliegenden Erfindung stellen Verbindungen der Formel (I) dar, in welcher R für Niederalkyl, besonders für Methyl steht, und worin $R_1$ und $R_2$ Triniederalkylmethylcarbonyl, worin Niederalkyl bis und mit 2 Kohlenstoffatome enthält, und $R_3$ Wasserstoff bedeuten, oder worin $R_1$ und $R_3$ Triniederalkylmethylcarbonyl, worin Niederalkyl bis und mit 2 Kohlenstoffatome enthält, und $R_2$ Wasserstoff bedeuten, sowie Salze, insbesondere pharmazeutisch verwendbare Salze davon, wobei diese Verbindungen als Gemische oder vorzugsweise in Form des vom anderen Isomeren praktisch freien Isomeren, vor allem in Form der entsprechenden 8-O,N-diacylierten Verbindung vorliegen.

In erster Linie betrifft die Erfindung die Verbindungen der Formel I, worin R Methyl ist, und $R_1$ und $R_2$ Pivaloyl und $R_3$ Wasserstoff bedeuten, oder $R_1$ und $R_3$ Pivaloyl und $R_2$ Wasserstoff bedeuten, und Salze, insbesondere pharmazeutisch verwendbare Salze davon, wobei diese Verbindungen als Gemisch oder in Form der individuellen Isomeren vorliegen; bevorzugt ist insbesondere das entsprechende 8-O,N-Dipivalyolderivat.

Die neuen Verbindungen der Formel I können in an sich bekannter Weise hergestellt werden, z.B. indem man eine Verbindung der Formel

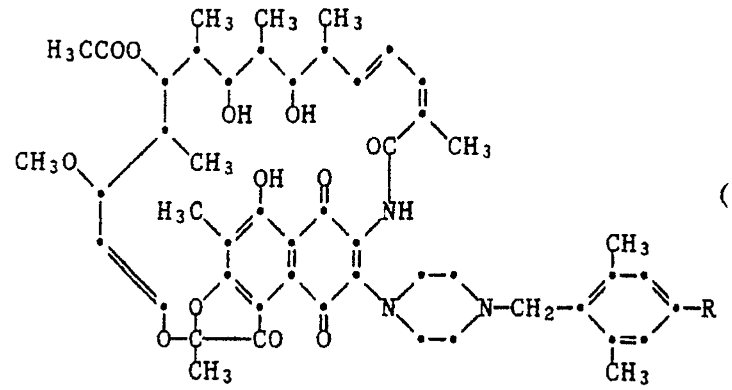

(II)

mit einem, einen Triniederalkylmethylcarbonylrest $R_1$ und $R_2$ bzw. $R_1$ und $R_3$ in die Stellung 8 und in die Stellung 21 oder in das Ringamidstickstoffatom einführenden Acylierungsmittel behandelt, und, wenn erwünscht, ein verfahrensgemäss erhältliches Gemisch von Isomeren auftrennt, und/oder ein verfahrensgemäss erhältliches Salz in die freie Verbindung oder in ein anderes Salz und/oder eine verfahrensgemäss erhältliche freie Verbindung in ein Salz überführt.

Die Einführung der Triniederalkylmethylcarbonylreste $R_1$ und $R_2$ bzw. $R_1$ und $R_3$ in die gewünschten Stellungen kann in an sich bekannnter Weise unter Verwendung eines üblichen, zur Einführung von solchen Resten geeigneten Acylierungsmittels erfolgen, wobei mindestens zwei Aequivalente des letzteren verwendet werden. Dabei kann man z.B. eine entsprechende Carbonsäure, wenn notwendig, in Gegenwart eines geeigneten Kondensationsmittels, wie Dicyclohexylcarbodiimid, vorzugsweise jedoch ein reaktionsfähiges Derivat einer solchen Carbonsäure, wie ein Anhydrid, in erster Linie ein gemischtes Anhydrid, wie dasjenige mit einer anorganischen Säure, wie einer Halogenwasserstoff-, besonders Chlor-, sowie Bromwasserstoffsäure (d.h. ein entsprechendes Säurehalogenid, z.B. -chlorid), oder mit einer organischen Säure, wie Trifluoressigsäure oder einem geeigneten Monoester der Kohlensäure, oder auch ein symmetrisches Anhydrid, ferner ein inneres Anhydrid, d.h. das entsprechende Keten, verwenden.

Das als Acylierungsmittel eingesetzte Derivat einer Carbonsäure wird vorzugsweise in Gegenwart eines basischen Mittels angewandt; als solches kommt in erster Linie eine nicht-acylierbare organische Base, wie eine heteroaromatische Base, z.B. Pyridin, Collidin oder Chinolin, tertiäres Amin, z.B. Triethylamin, N-Ethyl-piperidin, N-Methyl-morpholin oder 1,4-Dimethyl-piperazin, 1,5-Diazabicyclo-[5,4,0]undec-5-en in Frage.

Die Acylierungsreaktion wird üblicherweise in Gegenwart eines Lösungs- bzw. Verdünnungsmittels vorgenommen, wobei als solches auch ein Ueberschuss des Acylierungsmittels oder die zusammen mit einem Acylierungsmittel eingesetzte Base, z.B. Pyridin, dienen kann. Weitere Lösungsmittel, die z.B. auch im Gemisch mit einer Base verwendet werden können, sind z.B. nicht acylierbare organische Lösungsmittel, wie Kohlenwasserstoffe, z.B. Pentan, Hexan oder Cyclohexan, halogenierte Kohlenwasserstoffe, z.B. Methylenchlorid oder Chloroform, Ether, z.B. Diethylether, Ethylenglykoldimethylether, Tetrahydrofuran oder Dioxan, Säureester, z.B. Ethylacetat, Säureamide, z.B. Acetamid oder Dimethylformamid.

Die Reaktion wird üblicherweise bei Raumtemperatur oder leicht erhöhten Temperaturen, z.B. bis etwa 70 °C, vorgenommen, wobei man, wenn notwendig, unter einer Inertgasatmosphäre arbeitet. Die Acylierungsbedingungen, insbesondere die Menge des angewandten Acylierungsmittels, das Reaktionsmedium, die Temperatur und die Reaktionszeit, sind so zu wählen, dass beide Acylgruppen eingeführt werden, wobei man vorzugsweise gemäss den in den Beispielen näher illustrierten Methoden vorgeht. Der Ablauf der Reaktion kann zweckmässig mittels üblicher analytischer Methoden, insbesondere mittels Dünnschichtchromatographie, verfolgt werden.

Die Aufarbeitung des Reaktionsproduktes aus dem verfahrensgemäss erhältlichen Reaktionsgemisch erfolgt in an sich bekannter Weise, z.B. durch Verdünnen mit Wasser, und/oder gegebenenfalls durch Neutralisieren oder leichtes Ansäuern (bis etwa pH = 3) mit einer wässrigen Säure, wie einer anorganischen oder organischen Säure, z.B. einer Mineralsäure oder, vorteilhafterweise, Zitronensäure, und Zugabe eines mit Wasser nicht-mischbaren Lösungsmittels, wie eines chlorierten Kohlenwasserstoffs, z.B. Chloroform oder Methylenchlorid, wobei das Reaktionsprodukt in die organische Phase übergeht, aus welcher es in üblicher Weise, z.B. durch Trocknen, Eindampfen des Lösungsmittels und Kristallisation und/oder Chromatographie des Rückstandes oder andere übliche Reinigungsmethoden in gereinigter Form erhalten werden kann.

Die obige Reaktion ergibt im allgemeinen ein Gemisch der beiden diacylierten Verbindungen, üblicherweise einen überwiegenden Anteil der 8-O,N-diacylierten Verbindung. Das Gemisch kann in an sich

bekannter Weise, z.B. mittels fraktionierter Kristallisation, Chromatographie, etc. in die gewünschten individuellen Diacyl-Verbindungen aufgetrennt werden.

Die Ausgangsstoffe der Formel II sind bekannt und können in an sich bekannter Weise hergestellt werden; es wird z.B. auf die PCT-Anmeldung Publ. Nr. WO 87/02361 verwiesen.

Die Verbindungen der vorliegenden Erfindung können Säureadditionssalze, in erster Linie pharmazeutisch verwendbare Säureadditionssalze mit anorganischen oder organischen Säuren bilden. Solche sind u.a. Halogen-, z.B. Chlor- und Bromwasserstoffsäuren, Schwefelsäure, Phosphorsäure, Salpetersäure oder Perchlorsäure, oder aliphatische, carbocyclische (insbesondere aromatische) oder heterocyclische Carbon- oder Sulfonsäuren, wie Ameisen-, Essig-, Propion-, Bernstein-, Glykol-, Milch-, Aepfel-, Wein-, Zitronen-, Fumar-, Malein-, Hydroxymalein-, Brenztrauben-, Phenylessig-, Benzoe-, p-Aminobenzoe-, Anthranil-, p-Hydroxybenzoe-, Salicyl-, p-Aminosalicyl- oder Embonsäure, ferner Methansulfon-, Ethansulfon-, Hydroxyethansulfon, Ethylendisulfon-, Halogenbenzolsulfon-, Toluolsulfon-, Naphthalinsulfonsäuren oder Sulfanilsäure, ferner Methionin, Tryptophan, Lysin oder Arginin, sowie Ascorbinsäure.

Die Bildung der Salze, sowie die Ueberführung von Salzen in die freien Verbindungen erfolgt in an sich bekannter Weise. So erhält man die Säureadditionssalze z.B. durch Behandeln mit einer zur Salzbildung geeigneter Säure, wie einer der oben genannten, während Salze durch Behandeln mit basischen Mitteln, wie anorganischen Hydroxiden, Carbonaten und Bicarbonaten, oder organischen Basen und Ionenaustauschern in die freien Verbindungen umgewandelt werden können. Diese Salze mit den obengenannten Säuren oder andere Salze, wie z.B. Oxalate oder Pikrate, können auch zur Reinigung der erhaltenen Verbindungen dienen, indem man die freien Verbindungen in Salze überführt, diese abtrennt und aus den Salzen wiederum die freien Verbindungen gewinnt. Infolge der engen Beziehung zwischen den Verbindungen in freier Form und in Form ihrer Salze sind im vorausgegangenen und nachfolgend unter den freien Verbindungen sinn-und zweckgemäss gegebenenfalls auch die entsprechenden Salze zu verstehen.

Die Erfindung betrifft auch diejenigen Ausführungsformen des Verfahrens, nach denen man einen Ausgangsstoff in Form eines Derivates, z.B. Salzes, verwendet oder unter den Reaktionsbedingungen bildet.

Bei den Verfahren der vorliegenden Erfindung werden vorzugsweise solche Ausgangsstoffe verwendet, welche zu den eingangs als besonders wertvoll geschilderten Verbindungen führen.

Die vorliegende Erfindung umfasst auch die Verwendung der Verbindungen der Formel I und ihrer Salze allein oder zusammen mit Hilfsstoffen, sowie auch in Kombination mit anderen Wirkstoffen, als Mittel zur therapeutischen, und zwar sowohl kurativen wie auch präventiven Behandlung von Krankheiten oder krankhaften Zuständen, die z.B. durch einen erhöhten Gehalt an Cholesterin und/oder Triglyceriden im Blut, insbesondere im Blutserum, angezeigt oder verursacht werden. Die erfindungsgemässen Wirkstoffe werden in therapeutisch wirksamen Mengen, vorzugsweise in Form von pharmazeutischen Zusammensetzungen zusammen mit konventionellen pharma zeutischen Trägermaterialien und/oder Hilfsstoffen an den behandlungsbedürftigen Warmblüter, in erster Linie Menschen, verabreicht. Dabei werden z.B. an Warmblüter je nach Spezies, Körpergewicht, Alter und individuellem Zustand, sowie je nach Applikationsweise und insbesondere auch je nach individuellem Zustand, tägliche Dosen entsprechend etwa 1 bis etwa 100, insbesondere etwa 3 bis etwa 50 mg pro kg Körpergewicht, die in schweren Fällen überschritten werden können, verabreicht. Sinngemäss umfasst die Erfindung auch die entsprechende Methode zur medizinischen Behandlung.

Die Erfindung betrifft im weiteren pharmazeutische Zusammensetzungen, welche die Verbindungen der vorliegenden Erfindung als Wirkstoffe enthalten, sowie Verfahren zu ihrer Herstellung.

Bei den erfindungsgemässen pharmazeutischen Präparaten handelt es sich um solche zur enteralen, wie peroralen oder rektalen, sowie zur parenteralen Verabreichung an Warmblüter. Entsprechende Dosiseinheitsformen, insbesondere zur peroralen Verabreichung, z.B. Dragées, Tabletten oder Kapseln, enthalten vorzugsweise von etwa 50 bis etwa 500 mg, insbesondere von etwa 100 bis etwa 300 mg des Wirkstoffs zusammen mit pharmazeutisch verwendbaren Träger- und/oder Hilfsstoffen.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärkekleister zubereitet z.B. aus Mais-, Weizen-, Reis- oder Kartoffelstärke, Gelatine, Traganth, Methylcellulose und/oder, wenn erwünscht, Sprengmittel, wie die obengenannten Stärken, ferner Carboxymethylstärke, quervernetztes Polyvinylpyrrolidon, Agar, Alginsäure oder ein Salz davon, wie Natriumalginat. Hilfsmittel sind in erster Linie Fliessregulier-und Schmiermittel, z.B. Kieselsäure, Talk, Stearinsäure oder Salze davon, wie Magnesium- oder Calciumstearat, und/oder Polyethylenglykol. Dragée-kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinylpyrrolidon, Polyethylenglykol und/oder Titandioxid enthalten, oder Lacklösungen in geeigneten organischen Lösungsmitteln, oder, zur Herstellung von Magensaft-resistenten Ueberzügen, Lösungen von geeigneten

Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Weitere oral anwendbare pharmazeutische Präparate sind Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Lactose, Bindemitteln, wie Stärken und/oder Gleitmitteln, wie Talk oder Magnesiumstearat, und gegebenenfalls von Stabilisatoren enthalten. In weichen Kapseln ist der Wirkstoff vorzugsweise in geeigneten Flüssigkeiten, wie fetten Oelen, Paraffinöl oder flüssigen Polyethylenglykolen, gelöst oder suspendiert, wobei gegebenenfalls Stabilisatoren zugefügt sein können.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Die pharmazeutischen Präparate der vorliegenden Erfindung können in an sich bekannter Weise, z.B. mittels konventioneller Misch-, Granulier-, Dragier-, Lösungs- oder Lyophilisierungsverfahren, hergestellt werden. So kann man pharmazeutische Präparate zur oralen Anwendung erhalten, indem man den Wirkstoff mit festen Hilfsstoffen kombiniert, ein erhaltenes Gemisch gegebenenfalls granuliert, und das Gemisch bzw. Granulat, wenn erwünscht oder notwendig, nach Zugabe von geeigneten Hilfsstoffen, zu Tabletten oder Dragée-Kernen verarbeitet.

Die nachfolgenden Beispiele illustrieren die oben beschriebene Erfindung, schränken jedoch deren Umfang in keiner Weise ein. Temperaturen werden in Celsiusgraden angegeben.

Beispiel 1: 8-O,N- und 8-O,21-O-Dipivaloyl-3-[4-(2,4,6-trimethylbenzyl)-1-piperazinyl]-rifamycin S

Eine Lösung von 25 g 3-[4-(2,4,6-trimethylbenzyl)-1-piperazinyl]-rifamycin S in 250 ml Pyridin wird mit 35,5 ml Pivaloylchlorid (10,5 Aequivalente) tropfenweise versetzt und 8 Std. bei Raumtemperatur gerührt, bis im Dünnschichtchromatogramm kein Ausgangsmaterial mehr nachgewiesen werden kann. Anschliessend wird die Lösung mit 150 ml Methanol versetzt, 1 Std. bei Raumtemperatur gerührt, um überschüssiges Pivaloylchlorid zu zersetzen, und zur Trockne eingedampft. Der Rückstand wird in 300 ml Methylenchlorid aufgenommen, die Lösung filtriert, und das Filtrat mit 300 ml Wasser extrahiert. Die wässrige Phase wird mit 1N Salzsäure auf pH 3 bis 4 eingestellt und dreimal mit Methylenchlorid extrahiert. Die vereinigten Methylenhloridextrakte werden dreimal mit je 100 ml Wasser gewaschen, getrocknet und zur Trockne eingedampft. Der Rückstand wird auf eine Säule aus 1000 g Silicagel aufgetragen und mit einem 1:4-Gemisch von Essigsäureethylester und Cyclohexan eluiert. Die ersten Fraktionen enthalten das 8-O,21-O-Dipivaloyl-3-[4-(2,4,6-trimethylbenzyl)-1-piperazinyl]-rifamycin S, das durch weitere Chromatographie an Silicagel und Kristallisation aus Diethylether in Form von violettbraunen Kristallen vom Smp. 135-145° C (Zersetzung) erhalten wird. Die nachfolgenden Fraktionen enthalten als Hauptprodukt der Reaktion das 8-O,N-Dipivaloyl-3-[4-(2,4,6-trimethylbenzyl)-1-piperazinyl]-rifamycin S, welches, aus Diethylether kristallisiert, rot-violette Kristalle vom Smp. 157-163° C bildet.

Physikalische Kenndaten:

a) 8-O,21-O-Dipivaloyl-Verbindung:

Ultraviolettabsorptionsspektrum
in Ethanol) [$\lambda_{max}(\epsilon)$]: 218 (36400), 258 (Schulter), 318 (Schulter), 354 (8800), 517 (1800) nm.
Massenspektrum: m/z = 1080 ($M^+$ + H) entsprechend der Summenformel $C_{61}H_{81}N_3O_{14}$.
$^1$H-NMR (in $CDCl_3$) [ppm (Zuordnung)]:
6,83 s (2H am aromatischen Ring des Benzylrestes)
5,2 dd (H-21)

2,37 } s (aromatisch gebundene Methylgruppen)
2,27 }

3,48 AB (Methylen des Benzylrestes)

1,47 } s (Methylgruppen der Pivaloylreste)
1,42 }

0,95 } s (4H von Methylgruppen oder Ansaring)
0,85 }
0,70 }
0,18 }

$^{13}$C-NMR (in $CDCl_3$) [ppm (Zuordnung)]:
179,2 1C (Pivaloyl-carbonyl)
176,8 1C (Pivaloyl-carbonyl)

27,3 3C } (Methylgruppen der Pivaloylreste)
27,2 3C }

53,2 2C } (Methylengruppen des Piperazinrestes)
49,0 2C }

56,0 1C (Methylen des Benzylrestes)

173,5 2C } (Trimethylphenyl)
135,6 1C }
131,1 1C }
128,6 2C }
20,4 3C }

b) 8-O,N-Dipivaloyl-Verbindung:

Ultraviolettabsorptionsspektrum
(in Ethanol) [$\lambda_{max}(\epsilon)$]: 264 (31880), 320 (Schulter), 350 (Schulter), 524 (2200) nm.
Massenspektrum: m/z = 1080 ($M^+$ + H) entsprechend der Summenformel für $C_{61}H_{81}N_3O_{14}$.
Infrarotabsorptionsspektrum
(in $CH_2Cl_2$): 3500, 2960, 2940, 1735, 1710, 1640, 1600, 1565 $cm^{-1}$, kein Amid-NH- vorhanden.
$^1$H-NMR ($CDCl_3$) [ppm (Zuordnung)]:
6,83 s (2H vom aromatischen Ring des Benzylrestes)
3,45 AB (Methylen des Benzylrestes)
3,05 m (H-21)

2,33 } s (3 Methylgruppen am Benzylrest)
2,26

1,48 } s (6 Methylgruppen des Pivaloyls)
1,44

1,07
0,96 } d (4H von Methylgruppen oder Ansaring)
0,81
0,24

$^{13}$C-NHR (in $CDCl_3$):
190,4 1C (Pivaloyl-carbonyl)
179,0 1C (Pivaloyl-carbonyl)
45,3 1C (quaternäres C der Pivaloylreste)
40,5 1C (quaternäres C der Pivaloylreste)
29,4 3C (3 Methylgruppen der Pivaloylreste)
27,7 3C (3 Methylgruppen der Pivaloylreste)

54,9 1C (Methylen des Benzylrestes)

54,9 1C (Methylen des Benzylrestes)

137,5 2C
135,7 1C
131,3 1C } (2,4,6-Trimethylphenyl)
128,7 2C
19,7 3C

Das Ausgangsmaterial kann folgendermassen erhalten werden:

a) Eine Lösung von 50 g Rifamycin S in 500 ml Dioxan wird mit 30 g N-(2,4,6-Trimethylbenzyl)-piperazin versetzt und bei Raumtemperatur während 18 Stunden stehen gelassen. Anschliessend säuert man durch Zugabe von 10%iger wässriger Zitronensäurelösung an und nimmt das Reaktionsprodukt in Methylenchlorid auf. Nach dem Trocknen und Eindampfen des Methylenchloridextraktes wird der dunkel gefärbte Rückstand in Ethanol gelöst und tropfenweise mit wässeriger Ascorbinsäure versetzt. Das 3-[4-(2,4,6-Trimethylbenzyl)-1-piperazinyl]-rifamycin SV fällt in Form von gelben Kristallen vom Smp. 178-181 °C (teilweise Zersetzung) aus.

b) Eine Lösung von 10 g des gemäss a) hergestellten Produktes in 200 ml Methylenchlorid wird mit 10 g feinpulverisiertem Mangandioxid 5 Minuten intensiv gerührt. Die festen Anteile werden abfiltriert und das Filtrat zur Trockne eingedampft, womit das blauschwarze amorphe 3-[4-(2,4,6-Trimethylbenzyl)-1-piperazinyl)-rifamycin S resultiert, welches ohne weitere Reinigung weiterverarbeitet wird.

<u>Beispiel 2</u>: 8-O,N-Dipivaloyl-3-[4-(2,6-dimethyl-4-tert-butyl-benzyl)-1-piperazinyl]-rifamycin S.

In analoger Weise wie im Beispiel 1, ausgehend von 3-[4-(2,6-Dimethyl-4-tert-butyl-benzyl)-1-piperazinyl]-rifamycin S, wird die Titelverbindung als Feststoff ohne scharfen Schmelzpunkt erhalten; Massenspektrum: m/z = 1121 ($M^+$), entsprechend der Summenformel für $C_{64}H_{87}N_3O_{14}$;
Ultraviolettabsorptionsspektrum
(in Ethanol) [$\lambda_{max}$ ($\epsilon$)]: 263 (29960); (Schulter); 360 (Schulter); 526 (2000) nm;
Infrarotabsorptionsspektrum
(in Methylenchlorid): 3500, 2970, 1735, 1675, 1640, 1600, 1570 $cm^{-1}$.

$^1H$-NMR (in $CDCl_3$) [ppm (Zuordnung)]:
7,00 s (2H am aromatischen Ring des Benzylrestes)
3,43 AB (Methylen des Benzylrestes)
2,35 s (3 Methylgruppen am aromatischen Ring)

1,48 } s (6 Methylgruppen der Pivaloylreste)
1,44 }

Das Ausgangsmaterial kann folgendermassen hergestellt werden:

Eine Lösung von 5 g 3-Brom-rifamycin S in 50 ml Tetrahydrofuran wird mit 3 g N-(2,6-Dimethyl-4-tert-butyl-benzyl)-piperazin versetzt und bei 20° während 30 Minuten stehen gelassen. Anschliessend säuert man durch Zugabe von wässriger Zitronensäurelösung an und nimmt das Reaktionsprodukt in Methylenchlorid auf. Nach dem Trocknen und Eindampfen des Methylenchloridextraktes hinterbleibt ein dunkelgefärbter Rückstand; man löst ihn in Methanol und setzt tropfenweise wässrige Ascorbinsäure zu. Das 3-[4-(2,6-Dimethyl-4-tert-butyl-benzyl)-1-piperazinyl]-rifamycin SV fällt in Form von gelbgefärbten Kristallen aus, Smp. 260°. Durch Behandeln mit Mangandioxid, wie unter b) im Beispiel 1 beschrieben, erhält man das gewünschte Ausgangsmaterial der S-Reihe.

Beispiel 3:

In analoger Weise können folgende Verbindungen der Formel I erhalten werden:

8-O,21-O-Di-(2,2-dimethyl-butyryl)-3-[4-(2,4,6-trimethyl-benzyl)-1-piperazinyl]-rifamycin S und
8-O,N-Di-(2,2-dimethyl-butyryl)-3-[4-(2,4,6-trimethyl-benzyl)-1-piperazinyl]-rifamycin S oder ein Gemisch davon;
8-O,21-O-Di-(2-ethyl-2-methyl-butyryl)-3-[4-(2,4,6-trimethyl-benzyl)-1-piperazinyl]-rifamycin S und
8-O,N-Di-(2-ethyl-2-methyl-butyryl)-3-[4-(2,4,6-trimethyl-benzyl)-1-piperazinyl]-rifamycin S oder ein Gemisch davon;
8-O,21-O-Di-(2,2-diethyl-butyryl)-3-[4-(2,4,6-trimethyl-benzyl)-1-piperazinyl]-rifamycin S und
8-O,N-Di-(2,2-diethyl-butyryl)-3-[4(2,4,6-trimethyl-benzyl)-1-piperazinyl]-rifamycin S oder ein Gemisch davon;
8-O,21-O-Di-(2,2-dimethyl-valeryl)-3-[4-(2,4,6-trimethyl-benzyl)-1-piperazinyl]-rifamycin S und
8-O,N-Di-(2,2-dimethyl-valeryl)-3-[4-(2,4,6-trimethyl-benzyl)-1-piperazinyl]-rifamycin S oder ein Gemisch davon;
8-O,21-O-Di-(2-ethyl-2-methyl-valeryl)-3-[4-(2,4,6-trimethyl-benzyl)-1-piperazinyl]-rifamycin S und
8-O,N-Di-(2-ethyl-2-methyl-valeryl)-3-[4-(2,4,6-trimethyl-benzyl)-1-piperazinyl]-rifamycin S oder ein Gemisch davon;
8-O,21-O-Di-(2,2-diethyl-butyryl)-3-[4-(2,6-dimethyl-4-tert.-butyl-benzyl)-1-piperazinyl]-rifamycin S und
8-O,N-Di-(2,2-diethyl-butyryl)-3-[4-(2,6-dimethyl-4-tert.-butyl-benzyl)-1-piperazinyl]-rifamycin S oder ein Gemisch davon.

Beispiel 4:

Kapseln, enthaltend 250 mg 8-O,N-Dipivaloyl-3-[4-(2,4,6-trimethylbenzyl)-1-piperazinyl]-rifamycin S, können wie folgt hergestellt werden:

Zusammensetzung (für 1000 Kapseln):

| | |
|---|---|
| 8-O,N-Dipivaloyl-3-[4-(2,4,6-trimethylbenzyl)-1-piperazinyl]-rifamycin S | 250,0 g |
| Maisstärke | 50,0 g |
| Polyvinylpyrrolidon | 15,0 g |
| Magnesiumstearat | 5,0 g |
| Ethanol | q.s. |

Der Wirkstoff und die Maisstärke werden vermischt mit einer Lösung des Polyvinylpyrrolidons in 50 g

Ethanol befeuchtet. Die feuchte Masse wird durch ein Sieb mit einer Maschenweite von 3 mm gepresst und bei 45° getrocknet. Das trockene Granulat wird durch ein Sieb mit einer Maschenweite von 1 mm gesiebt und mit 5 g Magnesiumstearat vermischt. Die Mischung wird in Portionen von 0,320 g in Steckkapseln der Grösse 0 abgefüllt.

In analoger Weise kann auch das 8-O,21-O-Dipivaloyl-3-[4-(2,4,6-trimethylbenzyl)-1-piperazinyl]-rifamycin S oder ein Gemisch davon mit der entsprechenden 8-O,N-Dipivaloyl-Verbindung verwendet werden.

Beispiel 5:

250 g eines Wirkstoffes gemäss Beispiel 1 oder 2 und 1750 g fein geriebene Suppositoriengrundmasse (z.B. Kakaobutter) werden gründlich gemischt und dann geschmolzen. Aus der durch Rühren homogen gehaltenen Schmelze werden 1000 Suppositorien von 2 g gegossen. Sie enthalten je 250 mg Wirkstoff.

## Ansprüche

1. Verbindungen der Formel

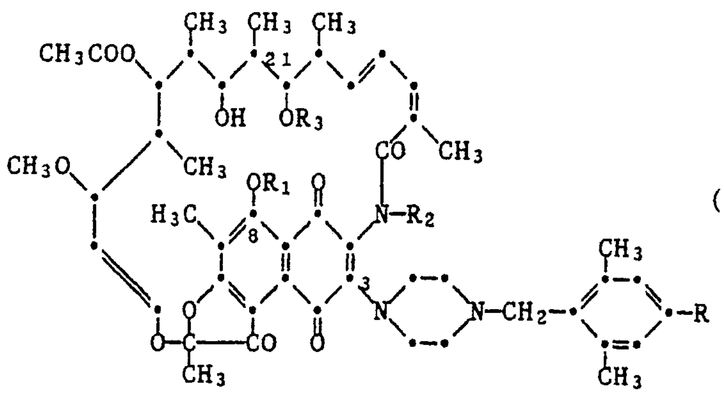

(I)

worin R Niederalkyl bedeutet, $R_1$ Triniederalkylmethylcarbonyl darstellt, und einer der Reste $R_2$ und $R_3$ Triniederalkylmethylcarbonyl und der andere Wasserstoff bedeuten, und Salze davon.

2. Verbindungen der Formel I gemäss Anspruch 1, in welcher R für Niederalkyl, besonders für Methyl steht, und worin $R_1$ und $R_2$ Triniederalkylmethylcarbonyl, worin Niederalkyl bis und mit 2 Kohlenstoffatome enthält, und $R_3$ Wasserstoff bedeuten, oder worin $R_1$ und $R_3$ Triniederalkylmethylcarbonyl, worin Niederalkyl bis und mit 2 Kohlenstoffatome enthält, und $R_2$ Wasserstoff bedeuten, und Salze davon.

3. Verbindungen der Formel I gemäss Anspruch 1, worin R Methyl ist, und $R_1$ und $R_2$ Pivaloyl und $R_3$ Wasserstoff bedeuten, oder $R_1$ und $R_3$ Pivaloyl und $R_2$ Wasserstoff bedeuten, und Salze davon.

4. Als Verbindung der Formel I gemäss Anspruch 1 das 8-O,21-O-Dipivaloyl-3-[4-(2,4,6-trimethylbenzyl)-1-piperazinyl]-rifamycin S und Salze davon.

5. Als Verbindung der Formel I gemäss Anspruch 1 das 8-O,N-Dipivaloyl-3-[4-(2,4,6-trimethylbenzyl)-1-piperazinyl]-rifamycin S und Salze davon.

6. Pharmazeutisch verwendbare Salze der Verbindungen der Formel I gemäss einem der Ansprüche 1-5.

7. Die Verbindungen der Ansprüche 1-6 als Pharmazeutika, insbesondere als Hypolipidämika.

8. Pharmazeutische Präparate enthaltend eine der Verbindungen gemäss einem der Ansprüche 1-6.

9. Verwendung der Verbindungen der Ansprüche 1-6 als Pharmazeutika, insbesondere als Hypolipidämika.

10. Verfahren zur Herstellung der Verbindungen der Formel I gemäss Anspruch 1, dadurch gekennzeichnet, dass man eine Verbindung der Formel

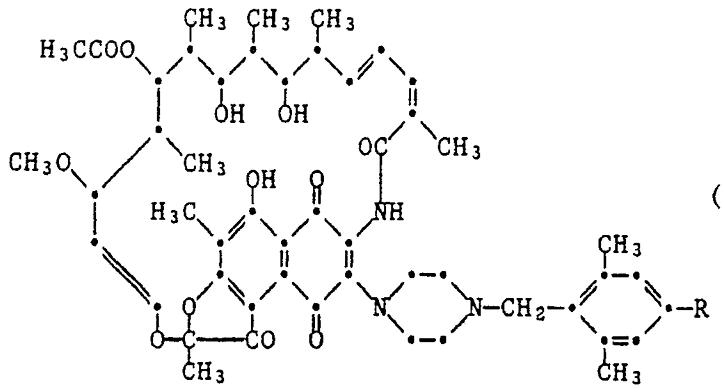

(II)

mit einem, einen Triniederalkylmethylcarbonylrest $R_1$ und $R_2$ bzw. $R_1$ und $R_3$ in die Stellung 8 und in die Stellung 21 oder in das Ringamidstickstoffatom einführenden Acylierungsmittel behandelt, und, wenn erwünscht, ein verfahrensgemäss erhältliches Gemisch von Isomeren auftrennt, und/oder ein verfahrensgemäss erhältliches Salz in die freie Verbindung oder in ein anderes Salz und/oder eine verfahrensgemäss erhältliche freie Verbindung in ein Salz überführt.

11. Die nach dem Verfahren des Anspruchs 10 erhältlichen Verbindungen.

Patentansprüche für die folgenden Vertragsstaaten: AT, ES, GR

1. Verfahren zur Herstellung von Verbindungen der Formel

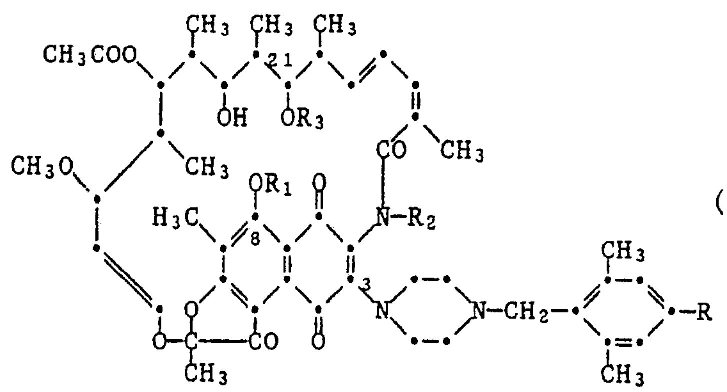

(I)

worin R Niederalkyl bedeutet, $R_1$ Triniederalkylmethylcarbonyl darstellt, und einer der Reste $R_2$ und $R_3$ Triniederalkylmethylcarbonyl und der andere Wasserstoff bedeuten, und Salzen davon, dadurch gekennzeichnet, dass man eine Verbindung der Formel

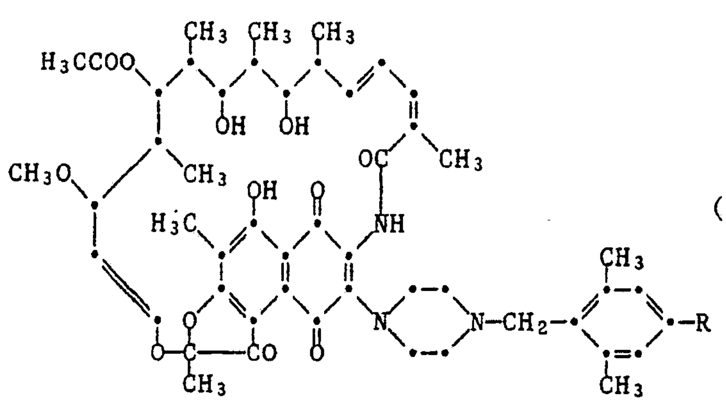

(II)

mit einem, einen Triniederalkylmethylcarbonylrest $R_1$ und $R_2$ bzw. $R_1$ und $R_3$ in die Stellung 8 und in die

Stellung 21 oder in das Ringamidstickstoffatom einführenden Acylierungsmittel behandelt, und, wenn erwünscht, ein verfahrensgemäss erhältliches Gemisch von Isomeren auftrennt, und/oder ein verfahrensgemäss erhältliches Salz in die freie Verbindung oder in ein anderes Salz und/oder eine verfahrensgemäss erhältliche freie Verbindung in ein Salz überführt.

2. Verfahren nach Anspruch 1, dadurch gekennzeichnet, dass eine, einem Triniederalkylmethylcarbonylrest entsprechende Carbonsäure oder ein reaktionsfähiges Derivat davon verwendet wird.

3. Verfahren nach Anspruch 2, dadurch gekennzeichnet, dass man ein entsprechendes Anhydrid, insbesondere ein gemischtes Anhydrid der Carbonsäure verwendet.

4. Verfahren nach Anspruch 3, dadurch gekennzeichnet, dass man ein entsprechendes Säurehalogenid, besonders Säurechlorid verwendet.

5. Verfahren nach einem der Ansprüche 1-4, dadurch gekennzeichnet, dass man Verbindungen der Formel I gemäss Anspruch 1, in welcher R für Niederalkyl, besonders für Methyl steht, und worin $R_1$ und $R_2$ Triniederalkylmethylcarbonyl, worin Niederalkyl bis und mit 2 Kohlenstoffatome enthält, und $R_3$ Wasserstoff bedeuten, oder worin $R_1$ und $R_3$ Triniederalkylmethylcarbonyl, worin Niederalkyl bis und mit 2 Kohlenstoffatome enthält, und $R_2$ Wasserstoff bedeuten, und Salze davon herstellt.

6. Verfahren nach einem der Ansprüche 1-4, dadurch gekennzeichnet, dass man Verbindungen der Formel I gemäss Anspruch 1, worin R Methyl ist, und $R_1$ und $R_2$ Pivaloyl und $R_3$ Wasserstoff bedeuten, oder $R_1$ und $R_3$ Pivaloyl und $R_2$ Wasserstoff bedeuten, und Salze davon herstellt.

7. Verfahren nach einem der Ansprüche 1-4, dadurch gekennzeichnet, dass man 8-O,21-O-Dipivaloyl-3-[4-(2,4,6-trimethylbenzyl)-1-piperazinyl]-rifamycin S und Salze davon herstellt.

8. Verfahren nach einem der Ansprüche 1-4, dadurch gekennzeichnet, dass man das 8-O,N-Dipivaloyl-3-[4-(2,4,6-trimethylbenzyl)-1-piperazinyl]-rifamycin S und Salze davon herstellt.

9. Verfahren zur Herstellung von pharmazeutischen Präparaten, dadurch gekennzeichnet, dass man eine Verbindung der Formel I gemäss Anspruch 1, worin R, $R_1$, $R_2$ und $R_3$ die im Anspruch 1 gegebenen Bedeutungen haben, oder ein Salz davon, gegebenenfalls zusammen mit Trägermaterialien, zu pharmazeutischen Präparaten verarbeitet.

Europäisches Patentamt

# EUROPÄISCHER RECHERCHENBERICHT

Nummer der Anmeldung

EP 87 81 0559

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| D,X | WO-A-8 702 361 (CIBA-GEIGY)<br>* Ansprüche 1,8 *<br>----- | 1,6 | C 07 D 498/08<br>A 61 K 31/395//<br>(C 07 D 498/08<br>C 07 D 307:00<br>C 07 D 267:00 ) |
| | | | **RECHERCHIERTE SACHGEBIETE (Int. Cl.4)** |
| | | | C 07 D 498/00<br>A 61 K 31/00 |

Der vorliegende Recherchenbericht wurde für alle Patentansprüche erstellt

| Recherchenort | Abschlußdatum der Recherche | Prüfer |
|---|---|---|
| DEN HAAG | 25-05-1988 | ALFARO I. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)